(19) Europäisches Patentamt
European Patent Office
Office européen des brevets

(11) Veröffentlichungsnummer: **0 436 464 A2**

(12) **EUROPÄISCHE PATENTANMELDUNG**

(21) Anmeldenummer: **90810976.2**

(22) Anmeldetag: **12.12.90**

(51) Int. Cl.[5]: **C08K 5/13**, C08K 5/3437, C08K 5/45, C08K 5/00, C08K 5/34, C07D 335/16, // (C08K5/00, 5:13, 5:34), (C08K5/00, 5:15, 5:34), (C08K5/00, 5:34, 5:45), (C08K5/34, 5:34, 5:3437)

(30) Priorität: **21.12.89 CH 4594/89**

(43) Veröffentlichungstag der Anmeldung: **10.07.91 Patentblatt 91/28**

(84) Benannte Vertragsstaaten: **DE FR GB IT**

(71) Anmelder: **CIBA-GEIGY AG**
**Klybeckstrasse 141**
**CH-4002 Basel (CH)**

(72) Erfinder: **Berner, Godwin, Dr.**
**Sommerhalde 5**
**CH-4102 Binningen (CH)**
Erfinder: **Valet, Andreas, Dr.**
**Im Bruckacker 18**
**W-7859 Eimeldingen (DE)**

(54) **UV-Absorber enthaltendes lichtempfindliches organisches Material.**

(57) Verbindungen der Formel

(1)

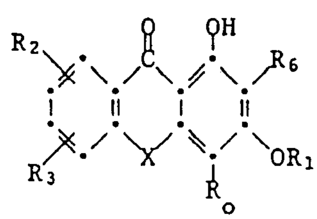

worin $R_o$, $R_1$, $R_2$, $R_3$, $R_6$ und X die in Anspruch 1 angegebene Bedeutung haben, eignen sich als UV-Absorber zur Verwendung in lichtempfindlichen organischen Materialien.

EP 0 436 464 A2

Jouve, 18, rue Saint-Denis, 75001 PARIS

# UV-ABSORBER ENTHALTENDES LICHTEMPFINDLICHES ORGANISCHES MATERIAL

Die vorliegende Erfindung betrifft neue UV-Absorber enthaltendes lichtempfindliches organisches Material sowie neue Thioxanthonverbindungen und ihre Verwendung als UV-Absorber.

In CH-A-379,760 wird ein Verfahren zum Schützen lichtempfindlicher Materialien vor der schädlichen Einwirkung von Licht, insbesondere von UV-Strahlen, beschrieben, worin 1-Hydroxyxanthonverbindungen Anwendung finden. Es wurde nun gefunden, dass auch gewisse Anthrone, Acridone und Thioxanthone mit gutem Erfolg für diesen Zweck eingesetzt werden können.

Gegenstand der vorliegenden Erfindung ist lichtempfindliches organisches Material, das als UV-Absorber mindestens eine Verbindung der Formel

(1) 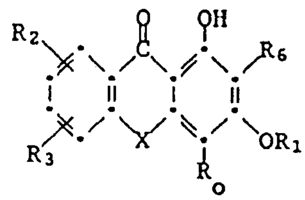

enthält, worin

X $CH_2$, NH oder S,

$R_o$ Wasserstoff oder ein Rest der Formel $-(CH_2)_nCO_2R$, worin n 1 oder 2 und R Alkyl mit 1 bis 18 Kohlenstoffatomen oder $-(CH_2CH_2O)_m$ H ist, worin m 1 bis 12 ist,

$R_1$ Wasserstoff, Alkyl mit 1 bis 12 Kohlenstoffatomen, mit Hydroxyl substituiertes und/oder mit Sauerstoff unterbrochenes Alkyl mit 2 bis 18 Kohlenstoffatomen, Alkenyl mit 2 bis 12 Kohlenstoffatomen, $-COR_4$, worin $R_4$ Alkyl mit 1 bis 12 Kohlenstoffatomen, Alkenyl mit 2 bis 12 Kohlenstoffatomen,

$-(CH_2)_y-\overset{O}{\overset{\|}{C}}R_5$ oder $-O-CH_2CH(OH)CH_2O-\overset{O}{\overset{\|}{C}}R_5$ ist, oder $R_1$ $-(CH_2)_yO-\overset{O}{\overset{\|}{C}}R_5$ ist,

worin $R_5$ Alkyl mit 1 bis 12 Kohlenstoffatomen oder Alkenyl mit 2 bis 12 Kohlenstoffatomen und y 1 bis 12 ist,

$R_2$ und $R_3$ unabhängig voneinander Wasserstoff, Alkyl mit 1 bis 12 Kohlenstoffatomen, Alkenyl mit 2 bis 12 Kohlenstoffatomen, $-OR_1$, worin $R_1$ die angegebene Bedeutung hat, oder Chlor sind, und

$R_6$ Wasserstoff oder Alkyl mit 1 bis 4 Kohlenstoffatomen ist.

Gegenstand der vorliegenden Erfindung sind ferner ein Verfahren zum Schützen lichtempfindlicher organischer Materialien vor UV-Strahlung durch Verwendung der Verbindungen der Formel (1), gegebenenfalls in Kombination mit sterisch gehinderten Aminen, Thioxanthonverbindungen der Formel (1) sowie ein Verfahren zu ihrer Herstellung.

In den Verbindungen der Formel (1), welche in den erfindungsgemässen Materialien enthalten sind, bedeutet der Substituent $R_o$ neben Wasserstoff einen Rest der Formel $-(CH_2)_n-CO_2R$. Darin ist R ein Alkylrest mit 1 bis 18 Kohlenstoffatomen, wie z.B. Methyl, Aethyl, Propyl, Butyl, Hexyl, Octyl, Decyl, Undecyl, Dodecyl, Hexadecyl und Octadecyl sowie entsprechende verzweigte Isomere, oder ein Rest der Formel $-(CH_2CH_2O)_m$ H, worin m eine ganze Zahl von 1 bis 12 ist. Index n ist 1 oder 2. $R_1$ ist neben Wasserstoff Alkyl mit 1 bis 18 Kohlenstoffatomen, also beispielsweise Methyl, Aethyl, Propyl, Butyl, Pentyl, Hexyl, Octyl, Decyl, Dodecyl, Hexadecyl oder Octadecyl, wobei auch entsprechende verzweigte Alkylreste in Frage kommen können. Alkylreste $R_1$ mit 2 bis 18 Kohlenstoffatomen können mit einer oder mehreren Hydroxylgruppen substituiert sein. Sie können auch durch ein oder mehrere Sauerstoffatome unterbrochen sein und gegebenenfalls zusätzlich eine oder mehrere Hydroxylgruppen aufweisen. Als Beispiele solcher Alkylreste wären Gruppierungen wie $-(CH_2)_x-O-(CH_2)_yH$ und $-(CH_2)_x-O-(CH_2)_y-O-(CH_2)_zH$, wobei die Summe von x und y bzw. von x, y und z 2 bzw. 3 bis 18 beträgt, und

$-\underset{OH}{C}H_2CHCH_2(O)_r(CH_2)_sH$ zu nennen, worin r 0 oder 1 und s 1 bis 15 ist.

$R_1$ bedeutet ferner Alkenyl mit 2 bis 12 Kohlenstoffatomen wie Aethenyl, Propenyl, Butenyl, Hexenyl, Octenyl, Nonenyl und Dodecenyl, wobei die Alkenylreste $R_1$ auch mehrfach ungesättigt sein können. Auch kann $R_1$ für die entsprechenden verzweigten Isomeren stehen.

Bedeutet $R_1$ einen Rest der Formel $—COR_4$, so ist $R_4$ Alkyl mit 1 bis 12 Kohlenstoffatomen, Alkenyl mit 2 bis 12 Kohlenstoffatomen, wobei Beispiele entsprechender Reste oben aufgeführt sind, ferner

$-(CH_2)_y-\overset{O}{\overset{\|}{C}}R_5$, oder $-O-CH_2CH(OH)CH_2O-\overset{O}{\overset{\|}{C}}R_5$, worin $R_5$ Alkyl mit 1 bis 12 Kohlenstoffatomen oder Alkenyl mit 2 bis 12 Kohlenstoffatomen (Beispiele siehe oben) und y 1 bis 12 ist.

Des weiteren ist $R_1$ $-(CH_2)_yO-\overset{O}{\overset{\|}{C}}R_5$, worin $R_5$ und y die angegebene Bedeutung haben.

Die Substituenten $R_2$ und $R_3$ bedeuten unabhängig voneinander Wasserstoff, Alkyl mit 1 bis 12 Kohlenstoffatomen oder Alkenyl mit 2 bis 12 Kohlenstoffatomen (Beispiele siehe oben), $—OR_1$, worin $R_1$ die angegebene Bedeutung hat, oder Chlor.

$R_6$ ist neben Wasserstoff Alkyl mit 1 bis 4 Kohlenstoffatomen wie Methyl, Propyl, Isopropyl, Butyl oder t-Butyl.

Die zweiwertige Gruppe X bedeutet $CH_2$, NH oder S. Werden die Verbindungen der Formel (1) in Kombination mit sterisch gehinderten Aminen oder Hydroxyphenylbenztriazolderivaten verwendet, kann X zusätzlich 0 bedeuten.

Vorzugsweise sind in den Verbindungen der Formel (1) die Substituenten $R_2$ und $R_3$ Wasserstoff.

Bevorzugt sind ferner Verbindungen der Formel (1), worin $R_o$ $—CH_2CH_2CO_2R$ ist, worin R Alkyl mit 1 bis 18 Kohlenstoffatomen oder $(CH_2CH_2O)_mH$ ist, worin m 4 bis 8 ist, sowie solche Verbindungen, worin $R_o$ ein Rest der Formel $—CH_2CH_2CO_2CH_3$ ist.

Eine weitere Gruppe bevorzugt verwendeter Verbindungen der Formel (1) ist dadurch gekennzeichnet, dass der Substituent $R_1$ Wasserstoff, Alkyl mit 4 bis 8 Kohlenstoffatomen, mit Hydroxyl substituiertes und/oder Sauerstoff unterbrochenes Alkyl mit 2 bis 12 Kohlenstoffatomen, Alkenyl mit 4 bis 12 Kohlenstoffatomen oder $—COR_4$ ist, worin $R_4$ Alkyl mit 4 bis 8 Kohlenstoffatomen ist.

Hiervon sind besonders solche Verbindungen geeignet, worin der Substituent $R_1$ Wasserstoff, Alkyl oder Alkenyl mit je 4 bis 8 Kohlenstoffatomen ist.

Lichtempfindliche organische Materialien können vor dem schädigenden Einfluss von UV-Strahlung geschützt werden, indem man diese Materialien mit einer Schutzschicht, z.B. einem Lack, versieht, die mindestens eine Verbindung der Formel (1) enthält, oder eine solche Verbindung auf an sich übliche Weise in das organische Material einarbeitet. Mit gutem Erfolg können zu diesem Zweck auch Kombinationen solcher Verbindungen der Formel (1), worin X $CH_2$, NH, O oder S ist, mit den bekannten Lichtschutzmitteln vom Typ der sterisch gehinderten Amine oder mit Hydroxyphenylbenztriazolderivaten verwendet werden.

Beispiele für die Vielfalt lichtempfindlicher organischer Materialien, die erfindungsgemäss vor schädigender Lichteinwirkung geschützt werden können, sind :

1. Polymere von Mono- und Diolefinen, beispielsweise Polypropylen, Polyisobutylen, Polybuten-1, Polymethylpenten-1, Polyisopren oder Polybutadien sowie Polymerisate von Cycloolefinen wie z.B. von Cyclopenten oder Norbornen ; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE).
2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).
3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie

Terpolymere von Ethylen mit Propylen und einem Dien, wie Hexadien, Dicyclopentadien oder Ethylidennorbornen ; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere und LLDPE/Ethylen-Acrylsäure-Copolymere.

3a. Kohlenwasserstoffharze (z.B. $C_5$-$C_9$) inklusive hydrierte Modifikationen davon (z.B. Klebrigmacherharze).

4. Polystyrol, Poly-(p-methylstyrol), Poly-(α-methylstyrol).

5. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Butadien-Alkylacrylat, Styrol-Maleinsäureanhydrid, Styrol-Acrylnitril-Methylacrylat ; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren ; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol oder α-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien ; Styrol, Acrylnitril und Methylmethacrylat auf Polybutadien ; Styrol und Maleinsäureanhydrid auf Polybutadien ; Styrol, Acrylnitril und Maleinsäureanhydrid oder Maleinsäureimid auf Polybutadien ; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.

7. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid ; sowie deren Copolymere, wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin ; sowie deren Copolymere mit in Punkt 1 genannten Olefinen.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxid, enthalten ; Polyacetale, die mit termoplastischen Polyurethanen, Acrylaten oder MBS modifiziert sind.

13. Polyphenylenoxide und -sulfide und deren Mischungen mit Styrolpolymeren oder Polyamiden.

14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, Polyamid 11, Polyamid 12, aromatische Polyamide ausgehend von m-Xylol, Diamin und Adipinsäure ; Polyamide, hergestellt aus Hexamethylendiamin und Iso-und/oder Terephthalsäure und gegebenenfalls einem Elastomer als Modifikator, z.B. Poly-2,4,4-trimethylhexamethylenterephthalamid, Poly-m-phenylen-isophthalamid. Block-Copolymere der vorstehend genannten Polyamide mit Polyolefinen, Olefin-Copolymeren, Ionomeren oder chemisch gebundenen oder gepfropften Elastomeren ; oder mit Polyethern, wie z.B. mit Polyethylenglykol, Polypropylenglykol oder Polytetramethylenglykol. Ferner mit EPDM oder ABS modifizierte Polyamide oder Copolyamide ; sowie während der Verarbeitung kondensierte Polyamide ("RIM-Polyamidsysteme").

16. Polyharnstoffe, Polyimide, Polyamid-imide und Polybenzimidazole.

17. Polyester, die sich von Dicarbonsäuren und Dialkoholen und/oder von Hydroxycarbonsäuren oder den

entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten ; ferner mit Polycarbonaten oder MBS modifizierte Polyester.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose ; sowie Kolophoniumharze und Derivate.

27. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

Als Beispiele für gehinderte Amine, die in Kombination mit den Verbindungen der Formel (1), worin X $CH_2$, NH, O oder S ist, verwendet werden können, sind Polyalkylpiperidinverbindungen zu nennen, die mindestens eine Gruppe der Formel

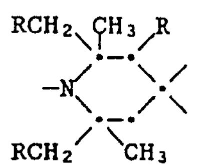

enthalten,

worin R Wasserstoff oder Methyl bedeutet. Bevorzugt ist R Wasserstoff. Es handelt sich dabei um Derivate von Polyalkylpiperidinen, insbesondere von 2,2,6,6-Tetramethylpiperidin. Bevorzugt tragen diese Verbindungen in 4-Stellung des Piperidinringes einen oder zwei polare Substituenten oder ein polares Spiro-Ringsystem. Es kann sich bei diesen Verbindungen um niedermolekulare oder oligomere oder polymere Verbindungen handeln.

Von Bedeutung sind insbesondere die folgenden Klassen von Polyalkylpiperidinen.

a) Verbindungen der Formel III,

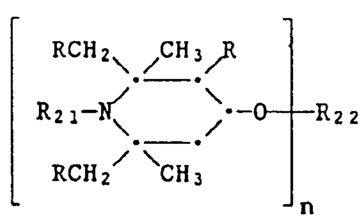

III

worin n eine Zahl von 1 bis 4, vorzugsweise 1 oder 2 bedeutet, R Wasserstoff oder Methyl bedeutet, $R_{21}$ Wasserstoff, Oxyl, Hydroxyl, $C_1$-$C_{12}$-Alkyl, $C_3$-$C_8$ Alkenyl, $C_3$-$C_8$-Alkinyl, $C_7$-$C_{12}$-Aralkyl, $C_1$-$C_{18}$-Alkoxy, $C_5$-$C_8$-Cycloalkoxy, $C_7$-$C_9$-Phenylalkoxy, $C_1$-$C_8$-Alkanoyl, $C_3$-$C_5$-Alkenoyl, $C_1$-$C_{18}$-Alkanoyloxy, Benzyloxy, Glycidyl oder eine Gruppe —$CH_2CH(OH)$-Z, worin Z Wasserstoff, Methyl oder Phenyl ist, bedeutet, wobei $R_{21}$ vorzugsweise H, $C_1$-$C_4$-Alkyl, Allyl, Benzyl, Acetyl oder Acryloyl ist und $R_{22}$, wenn n 1 ist, Wasserstoff, gegebenenfalls durch ein oder mehrere Sauerstoffatome unterbrochenes $C_1$-$C_{18}$-Alkyl, Cyanethyl, Benzyl, Glycidyl, einen einwertigen Rest einer aliphatischen, cycloaliphatischen, araliphatischen, ungesättigten oder aromatischen Carbonsäure, Carbaminsäure oder Phosphor enthaltenden Säure oder einen einwertigen Silylrest, vorzugsweise einen Rest einer aliphatischen Carbonsäure mit 2 bis 18 C-Atomen, einer cycloaliphatischen Carbonsäure mit 7 bis 15 C-Atomen, einer $\alpha,\beta$-ungesättigten Carbonsäure mit 3 bis 5 C-Atomen oder einer aromatischen Carbonsäure mit 7 bis 15 C-Atomen bedeutet, wenn n 2 ist, $C_1$-$C_{12}$-Alkylen, $C_4$-$C_{12}$-Alkenylen, Xylylen, einen zweiwertigen Rest einer aliphatischen, cycloaliphatischen, araliphatischen oder aromatischen Dicarbonsäure, Dicarbaminsäure oder Phosphor enthaltenden Säure oder einen zweiwertigen Silylrest, vorzugsweise einen Rest einer aliphatischen Dicarbonsäure mit 2 bis 36 C-Atomen, einer cycloaliphatischen oder aromatischen Dicarbonsäure mit 8-14 C-Atomen oder einer aliphatischen, cycloaliphatischen oder aromatischen Dicarbaminsäure mit 8-14 C-Atomen bedeutet, wenn n 3 ist, einen dreiwertigen Rest einer aliphatischen, cycloaliphatischen oder aromatischen Tricarbonsäure, einer aromatischen Tricarbaminsäure oder einer Phosphor enthaltenden Säure oder einen dreiwertigen Silylrest bedeutet und wenn n 4 ist, einen vierwertigen Rest einer aliphatischen, cycloaliphatischen oder aromatischen Tetracarbonsäure bedeutet.

Bedeuten etwaige Substituenten $C_1$-$C_{12}$-Alkyl, so stellen sie z.B. Methyl, Ethyl, n-Propyl, n-Butyl, sek.-Butyl, tert.-Butyl, n-Hexyl, n-Octyl, 2-Ethyl-hexyl, n-Nonyl, n-Decyl, n-Undecyl oder n-Dodecyl dar.

In der Bedeutung von $C_1$-$C_{18}$-Alkyl kann $R_{21}$ oder $R_{22}$ z.B. die oben angeführten Gruppen und dazu noch beispielsweise n-Tridecyl, n-Tetradecyl, n-Hexadecyl oder n-Octadecyl darstellen.

Wenn $R_{21}$ $C_3$-$C_8$-Alkenyl bedeutet, so kann es sich z.B. um 1-Propenyl, Allyl, Methallyl, 2-Butenyl, 2-Pentenyl, 2-Hexenyl, 2-Octenyl, 4-tert.-Butyl-2-butenyl handeln.

$R_{21}$ ist als $C_3$-$C_8$-Alkinyl bevorzugt Propargyl.

Als $C_7$-$C_{12}$-Aralkyl ist $R_{21}$ insbesondere Phenethyl und vor allem Benzyl.

$R_{21}$ ist als $C_1$-$C_8$-Alkanoyl beispielsweise Formyl, Propionyl, Butyryl, Octanoyl, aber bevorzugt Acetyl und als $C_3$-$C_5$-Alkenoyl insbesondere Acryloyl.

$R_{21}$ als $C_1$-$C_{18}$-Alkoxy is z.B. Hexyloxy, Heptyloxy, Octyloxy oder Decyloxy. $R_{21}$ als Cycloalkoxy ist vorzugsweise Cyclohexyloxy. $R_{21}$ als Phenylalkoxy ist vorzugsweise Benzyloxy. $R_{21}$ als Alkanoyloxy ist z.B. Acetyloxy, Butyroyloxy, Hexanoyloxy, Octanoyloxy, Decanoyloxy oder Stearoyloxy.

Bedeutet $R_{22}$ einen einwertigen Rest einer Carbonsäure, so stellt es beispielsweise einen Essigsäure-, Capronsäure-, Stearinsäure-, Acrylsäure-, Methacrylsäure-, Benzoe- oder $\beta$-(3,5-Di-tert.-butyl-4-hydroxyphenyl)-propionsäurerest dar.

Bedeutet $R_{22}$ einen zweiwertigen Rest einer Dicarbonsäure, so stellt es beispielsweise einen Malonsäure-, Bernsteinsäure-, Glutarsäure-, Adipinsäure-, Korksäure-, Sebacinsäure-, Maleinsäure-, Itaconsäure-, Phthalsäure-, Dibutylmalonsäure-, Dibenzylmalonsäure-, Butyl-(3,5-di-tert.-butyl-4-hydroxybenzyl)-malonsäure- oder Bicycloheptendicarbonsäurerest dar.

Stellt $R_{22}$ einen dreiwertigen Rest einer Tricarbonsäure dar, so bedeutet es z.B. einen Trimellitsäure-, Citronensäure- oder Nitrilotriessigsäurerest.

Stellt $R_{22}$ einen vierwertigen Rest einer Tetracarbonsäure dar, so bedeutet es z.B. den vierwertigen Rest von Butan-1,2,3,4-tetracarbonsäure oder von Pyromellitsäure.

Bedeutet $R_{22}$ einen zweiwertigen Rest einer Dicarbaminsäure, so stellt es beispielsweise einen Hexamethylendicarbaminsäure- oder einen 2,4-Toluylen-dicarbaminsäurerest dar.

Bevorzugt sind Verbindungen der Formel III, worin R Wasserstoff ist, $R_{21}$ Wasserstoff oder Methyl ist, n 1 ist und $R_{22}$ $C_1$-$C_{18}$-Alkyl ist oder n 2 ist und $R_{22}$ der Diacylrest einer aliphatischen Dicarbonsäure mit 4-12 C-Atomen ist.

Beispiele für Polyalkylpiperidin-Verbindungen dieser Klasse sind folgende Verbindungen :

1) 4-Hydroxy-2,2,6,6-tetramethylpiperidin
2) 1-Allyl-4-hydroxy-2,2,6,6-tetramethylpiperidin
3) 1-Benzyl-4-hydroxy-2,2,6,6-tetramethylpiperidin
4) 1-(4-tert.-Butyl-2-butenyl)-4-hydroxy-2,2,6,6-tetramethylpiperidin
5) 4-Stearoyloxy-2,2,6,6-tetramethylpiperidin
6) 1-Ethyl-4-salicyloyloxy-2,2,6,6-tetramethylpiperidin
7) 4-Methacryloyloxy-1,2,2,6,6-pentamethylpiperidin
8) 1,2,2,6,6-Pentamethylpiperidin-4-yl-$\beta$-(3,5-di-tert.-butyl-4-hydroxyphenyl)-propionat
9) Di-(1-benzyl-2,2,6,6-tetramethylpiperidin-4-yl)-maleinat
10) Di-(2,2,6,6-tetramethylpiperidin-4-yl)-succinat
11) Di-(2,2,6,6-tetramethylpiperidin-4-yl)-glutarat
12) Di-(2,2,6,6-tetramethylpiperidin-4-yl)-adipat
13) Di-(2,2,6,6-tetramethylpiperidin-4-yl)-sebacat
14) Di-(1,2,2,6,6-pentamethylpiperidin-4-yl)-sebacat
15) Di-(1,2,3,6-tetramethyl-2,6-diethyl-piperidin-4-yl)-sebacat
16) Di-(1-allyl-2,2,6,6-tetramethylpiperidin-4-yl)-phthalat

17) 1-Hydroxy-4-β-cyanoethyloxy-2,2,6,6-tetramethylpiperidin
18) 1-Acetyl-2,2,6,6-tetramethylpiperidin-4-yl-acetat
19) Trimellithsäure-tri-(2,2,6,6-tetramethylpiperidin-4-yl)-ester
20) 1-Acryloyl-4-benzyloxy-2,2,6,6-tetramethylpiperidin
21) Diethylmalonsäure-di(2,2,6,6-tetramethylpiperidin-4-yl)-ester
22) Dibutyl-malonsäure-di-(1,2,2,6,6-pentamethylpiperidin-4-yl)-ester
23) Butyl-(3,5-di-tert.-butyl-4-hydroxybenzyl)-malonsäure-di-(1,2,2,6,6-pentamethylpiperidin-4-yl)-ester
24) Di-(1-octyloxy-2,2,6,6-tetramethylpiperidin-4-yl)-sebacat
25) Di-(1-cyclohexyloxy-2,2,6,6-tetramethylpiperidin-4-yl)-sebacat
26) Hexan-1',6'-bis-(4-carbamoyloxy-1-n-butyl-2,2,6,6-tetramethylpiperidin)
27) Toluol-2',4'-bis-(4-carbamoyloxy-1-n-propyl-2,2,6,6-tetramethylpiperidin)
28) Tetra(2,2,6,6-tetramethylpiperidin-4-yl)-butan-1,2,3,4-tetracarboxylat
29) Tetra(1,2,2,6,6-pentamethylpiperidin-4-yl)-butan-1,2,3,4-tetracarboxylat
30) Tris-(1-propyl-2,2,6,6-tetramethylpiperidin-4-yl)-phosphit
31) Tris-(1-propyl-2,2,6,6-tetramethylpiperidin-4-yl)phosphat
32) Phenyl-[bis-(1,2,2,6,6-pentamethylpiperidin-4-yl)]-phosphonat
33) 4-Hydroxy-1,2,2,6,6-pentamethylpiperidin
34) 4-Hydroxy-N-hydroxyethyl-2,2,6,6-tetramethylpiperidin
35) 4-Hydroxy-N-(2-hydroxypropyl)-2,2,6,6-tetramethylpiperidin
36) 1-Glycidyl-4-hydroxy-2,2,6,6-tetramethylpiperidin

b) Verbindungen der Formel IV,

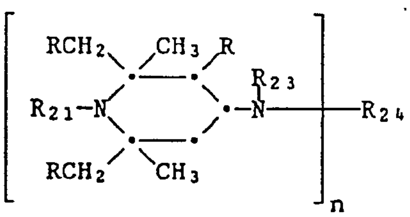

IV

worin n die Zahl 1 oder 2 bedeutet, R und $R_{21}$ die unter a) angegebene Bedeutung haben, $R_{23}$ Wasserstoff, $C_1$-$C_{12}$-Alkyl, $C_2$-$C_5$-Hydroxyalkyl, $C_5$-$C_7$-Cycloalkyl, $C_7$-$C_8$-Aralkyl, $C_2$-$C_{18}$-Alkanoyl, $C_3$-$C_5$-Alkenoyl, Benzoyl oder eine Gruppe der Formel

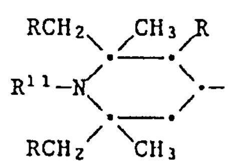

ist und $R_{24}$ wenn n 1 ist, Wasserstoff, $C_1$-$C_{18}$-Alkyl, $C_3$-$C_8$-Alkenyl, $C_5$-$C_7$-Cycloalkyl, mit einer Hydroxy-, Cyano-, Alkoxycarbonyl- oder Carbamidgruppe substituiertes $C_1$-$C_4$-Alkyl, Glycidyl, eine Gruppe der Formel —$CH_2$-CH(OH)—Z oder der Formel —CONH-Z ist, worin Z Wasserstoff, Methyl oder Phenyl bedeutet ; wenn n 2 ist, $C_2$-$C_{12}$-Alkylen, $C_6$-$C_{12}$-Arylen, Xylylen, eine —$CH_2$-CH(OH)-$CH_2$-Gruppe oder eine Gruppe —$CH_2$-CH(OH)-$CH_2$-O-D-O— bedeutet, worin D $C_2$-$C_{10}$-Alkylen, $C_6$-$C_{15}$-Arylen, $C_6$-$C_{12}$-Cycloalkylen ist, oder vorausgesetzt, dass $R_{23}$ nicht Alkanoyl, Alkenoyl oder Benzoyl bedeutet, $R_{24}$ auch einen zweiwertigen Rest einer aliphatischen, cycloaliphatischen oder aromatischen Dicarbonsäure oder Dicarbaminsäure oder auch die Gruppe —CO— bedeuten kann, oder $R_{23}$ und $R_{24}$ zusammen, wenn n 1 ist, den zweiwertigen Rest einer aliphatischen, cycloaliphatischen oder aromatischen 1,2- oder 1,3-Dicarbonsäure bedeuten können.

Stellen etwaige Substituenten $C_1$-$C_{12}$- oder $C_1$-$C_{18}$-Alkyl dar, so haben sie die bereits unter a) angegebene Bedeutung.

Bedeuten etwaige Substituenten $C_5$-$C_7$-Cycloalkyl, so stellen sie insbesondere Cyclohexyl dar.

Als $C_7$-$C_8$-Aralkyl ist $R_{23}$ insbesondere Phenylethyl oder vor allem Benzyl. Als $C_2$-$C_5$-Hydroxyalkyl ist $R_{23}$ insbesondere 2-Hydroxyethyl oder 2-Hydroxypropyl.

$R_{23}$ ist als $C_2$-$C_{18}$-Alkanoyl beispielsweise Propionyl, Butyryl, Octanoyl, Dodecanoyl, Hexadecanoyl, Octadecanoyl, aber bevorzugt Acetyl und als $C_3$-$C_5$-Alkenoyl insbesondere Acryloyl.

Bedeutet $R_{24}$ $C_2$-$C_8$-Alkenyl, dann handelt es sich z.B. um Allyl, Methallyl, 2-Butenyl, 2-Pentenyl, 2-Hexenyl oder 2-Octenyl.

$R_{24}$ als mit einer Hydroxy-, Cyano-, Alkoxycarbonyl- oder Carbamidgruppe substituiertes $C_1$-$C_4$-Alkyl kann z.B. 2-Hydroxyethyl, 2-Hydroxypropyl, 2-Cyanethyl, Methoxycarbonylmethyl, 2-Ethoxycarbonylethyl, 2-Aminocarbonylpropyl oder 2-(Dimethylaminocarbonyl)-ethyl sein.

Stellen etwaige Substituenten $C_2$-$C_{12}$-Alkylen dar, so handelt es sich z.B. um Ethylen, Propylen, 2,2-Dimethylpropylen, Tetramethylen, Hexamethylen, Octamethylen, Decamethylen oder Dodecamethylen.

Bedeuten etwaige Substituenten $C_6$-$C_{15}$-Arylen, so stellen sie z.B. o-, m-oder p-Phenylen, 1,4-Naphthylen oder 4,4'-Diphenylen dar.

Als $C_6$-$C_{12}$-Cycloalkylen ist D insbesondere Cyclohexylen.

Bevorzugt sind Verbindungen der Formel IV, worin n 1 oder 2 ist, R Wasserstoff ist, $R_{21}$ Wasserstoff oder Methyl ist, $R_{23}$ Wasserstoff, $C_1$-$C_{12}$-Alkyl oder eine Gruppe der Formel

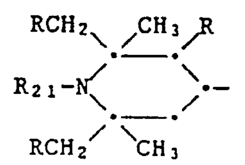

ist und $R_{24}$ im Fall von n = 1 Wasserstoff oder $C_1$-$C_{12}$-Alkyl ist, und im Fall von n = 2 $C_2$-$C_8$-Alkylen ist.

Beispiele für Polyalkylpiperidin-Verbindungen dieser Klasse sind folgende Verbindungen :

37) N,N'-Bis-(2,2,6,6-tetramethylpiperidin-4-yl)-hexamethylen-1,6-diamin
38) N,N'-Bis-(2,2,6,6-tetramethylpiperidin-4-yl)-hexamethylen-1,6-diacetamid
39) Bis-(2,2,6,6-tetramethylpiperidin-4-yl)-amin
40) 4-Benzoylamino-2,2,6,6-tetramethylpiperidin
41) N,N'-Bis-(2,2,6,6-tetramethylpiperidin-4-yl)-N,N'-dibutyl-adipamid
42) N,N'-Bis-(2,2,6,6-tetramethylpiperidin-4-yl)-N,N'-dicyclohexyl-2-hydroxypropylen-1,3-diamin
43) N,N'-Bis-(2,2,6,6-tetramethylpiperidin-4-yl)-p-xylylen-diamin
44) N,N'-Bis-(2,2,6,6-tetramethylpiperidin-4-yl)-succindiamid
45) N-(2,2,6,6-Tetramethylpiperidin-4-yl)-β-aminodipropionsäure-di-(2,2,6,6-tetramethylpiperidin-4-yl)-ester
46) Die Verbindung der Formel

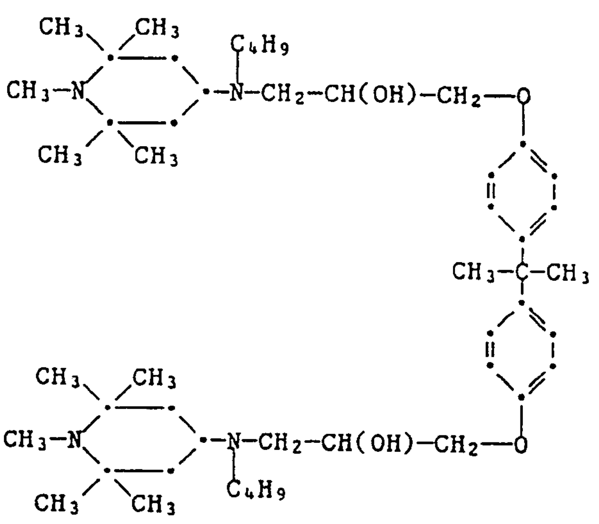

47) 4-(Bis-2-hydroxyethyl-amino)-1,2,2,6,6-pentamethylpiperidin
48) 4-(3-Methyl-4-hydroxy-5-tert.-butyl-benzoesäureamido)-2,2,6,6-tetramethylpiperidin
49) 4-Methacrylamido-1,2,2,6,6-pentamethylpiperidin
c) Verbindungen der Formel V,

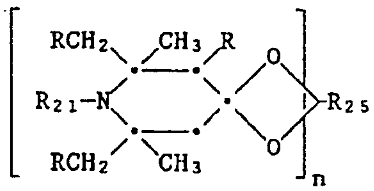
V

worin n die Zahl 1 oder 2 bedeutet, R und $R_{21}$ die unter a) angegebene Bedeutung haben und $R_{25}$, wenn n 1 ist, $C_2$-$C_8$-Alkylen oder -Hydroxyalkylen oder $C_4$-$C_{22}$-Acyloxyalkylen, wenn n 2 ist, die Gruppe $(-CH_2)_2C(CH_2-)_2$ bedeutet.

Bedeutet $R_{25}$ $C_2$-$C_8$-Alkylen oder -Hydroxyalkylen, so stellt es beispielsweise Ethylen, 1-Methyl-ethylen, Propylen, 2-Ethyl-propylen oder 2-Ethyl-2-hydroxymethylpropylen dar.

Als $C_4$-$C_{22}$-Acyloxyalkylen bedeutet $R_{25}$ z.B. 2-Ethyl-2-acetoxymethylpropylen.

Beispiele für Polyalkylpiperidin-Verbindungen dieser Klasse sind folgende Verbindungen :

50) 9-Aza-8,8,10,10-tetramethyl-1,5-dioxaspiro[5.5]undecan

51) 9-Aza-8,8,10,10-tetramethyl-3-ethyl-1,5-dioxaspiro[5.5]undecan

52) 8-Aza-2,7,7,8,9,9-hexamethyl-1,4-dioxaspiro[4.5]decan

53) 9-Aza-3-hydroxymethyl-3-ethyl-8,8,9,10,10-pentamethyl-1,5-dioxaspiro[5.5]undecan

54) 9-Aza-3-ethyl-3-acetoxymethyl-9-acetyl-8,8,10,10-tetramethyl-1,5-dioxaspiro[5.5]undecan

55) 2,2,6,6-Tetramethylpiperidin-4-spiro-2'-(1',3'-dioxan)-5'-spiro-5''-(1'',3''-dioxan)-2''-spiro-4'''-(2''',2''',6''',6'''-tetramethylpiperidin).

d) Verbindungen der Formeln VIA, VIB und VIC

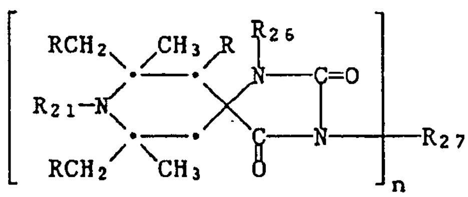
VIA

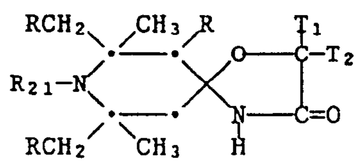
VIB

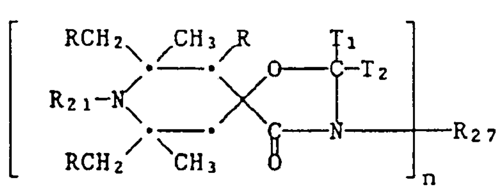
VIC

worin n die Zahl 1 oder 2 bedeutet, R und $R_{21}$ die unter a) angegebene Bedeutung haben, $R_{26}$ Wasserstoff, $C_1$-$C_{12}$-Alkyl, Allyl, Benzyl, Glycidyl oder $C_2$-$C_6$-Alkoxyalkyl ist und $R_{27}$, wenn n 1 ist, Wasserstoff, $C_1$-$C_{12}$-Alkyl, $C_3$-$C_5$-Alkenyl, $C_7$-$C_9$-Aralkyl, $C_5$-$C_7$ Cycloalkyl, $C_2$-$C_4$-Hydroxyalkyl, $C_2$-$C_6$-Alkoxyalkyl, $C_6$-$C_{10}$-Aryl, Glycidyl oder eine Gruppe der Formel $-(CH_2)_p$-COO-Q oder der Formel $-(CH_2)_p$-O-CO-Q ist, worin p 1 oder 2 und Q $C_1$-$C_4$ Alkyl oder Phenyl sind, wenn n 2 ist, $C_2$-$C_{12}$ Alkylen, $C_4$-$C_{12}$-Alkenylen, $C_6$-$C_{12}$ Arylen, eine Gruppe $-CH_2$-CH(OH)-$CH_2$-O-D-O-$CH_2$-CH(OH)-$CH_2-$, worin D $C_2$-$C_{10}$ Alkylen, $C_6$-$C_{15}$-Arylen, $C_6$-$C_{12}$ Cycloalkylen ist, oder eine Gruppe

$-CH_2CH(OZ')CH_2$-$(OCH_2$-$CH(OZ')CH_2)_2-$ bedeutet, worin Z' Wasserstoff, $C_1$-$C_{18}$-Alkyl, Allyl, Benzyl, $C_2$-$C_{12}$-Alkanoyl oder Benzoyl ist, $T_1$ und $T_2$ unabhängig voneinander Wasserstoff, $C_1$-$C_{18}$-Alkyl oder gegebenenfalls durch Halogen oder $C_1$-$C_4$-Alkyl substituiertes $C_6$-$C_{10}$-Aryl oder $C_7$-$C_9$-Aralkyl bedeuten oder $T_1$ und $T_2$ zusam-

men mit dem sie bindenden C-Atom einen $C_5$-$C_{12}$-Cycloalkanring bilden.

Bedeuten etwaige Substituenten $C_1$-$C_{12}$-Alkyl, so stellen sie z.B. Methyl, Ethyl, n-Propyl, n-Butyl, sek.-Butyl, tert.-Butyl, n-Hexyl, n-Octyl, 2-Ethyl-hexyl, n-Nonyl, n-Decyl, n-Undecyl oder n-Dodecyl dar.

Etwaige Substituenten in der Bedeutung von $C_1$-$C_{18}$-Alkyl können z.B. die oben angeführten Gruppen und dazu noch beispielsweise n-Tridecyl, n-Tetradecyl, n-Hexadecyl oder n-Octadecyl darstellen.

Bedeuten etwaige Substituenten $C_2$-$C_6$-Alkoxyalkyl, so stellen sie z.B. Methoxymethyl, Ethoxymethyl, Propoxymethyl, tert.-Butoxymethyl, Ethoxyethyl, Ethoxypropyl, n-Butoxyethyl, tert.-Butoxyethyl, Isopropoxyethyl oder Propoxypropyl dar.

Stellt $R_{27}$ $C_3$-$C_5$-Alkenyl dar, so bedeutet es z.B. 1-Propenyl, Allyl, Methallyl, 2-Butenyl oder 2-Pentenyl.

Als $C_7$-$C_9$-Aralkyl sind $R_{27}$, $T_1$ und $T_2$ insbesondere Phenethyl oder vor allem Benzyl. Bilden $T_1$ und $T_2$ zusammen mit dem C-Atom einen Cycloalkanring, so kann dies z.B. ein Cyclopentan-, Cyclohexan-, Cyclooctan-oder Cyclododecanring sein.

Bedeutet $R_{27}$ $C_2$-$C_4$-Hydroxyalkyl, so stellt es z.B. 2-Hydroxyethyl, 2-Hydroxypropyl, 2-Hydroxybutyl oder 4-Hydroxybutyl dar.

Als $C_6$-$C_{10}$-Aryl bedeuten $R_{27}$, $T_1$ und $T_2$ insbesondere Phenyl, α- oder β-Naphthyl, die gegebenenfalls mit Halogen oder $C_1$-$C_4$-Alkyl substituiert sind.

Stellt $R_{27}$ $C_2$-$C_{12}$-Alkylen dar, so handelt es sich z.B. um Ethylen, Propylen, 2,2-Dimethylpropylen, Tetramethylen, Hexamethylen, Octamethylen, Decamethylen oder Dodecamethylen.

Als $C_4$-$C_{12}$-Alkenylen bedeutet $R_{27}$ insbesondere 2-Butenylen, 2-Pentenylen oder 3-Hexenylen.

Bedeutet $R_{27}$ $C_6$-$C_{12}$ Arylen, so stellt es beispielsweise o-, m- oder p-Phenylen, 1,4-Naphthylen oder 4,4'-Diphenylen dar.

Bedeutet Z' $C_2$-$C_{12}$ Alkanoyl, so stellt es beispielsweise Propionyl, Butyryl, Octanoyl, Dodecanoyl, aber bevorzugt Acetyl dar.

D hat als $C_2$-$C_{10}$ Alkylen, $C_6$-$C_{15}$ Arylen oder $C_6$-$C_{12}$ Cycloalkylen die unter b) angegebene Bedeutung.

Beispiele für Polyalkylpiperidin-Verbindungen dieser Klasse sind folgende Verbindungen :

56) 3-Benzyl-1,3,8-triaza-7,7,9,9-tetramethylspiro[4.5]decan-2,4-dion
57) 3-n-Octyl-1,3,8-triaza-7,7,9,9-tetramethylspiro[4.5]decan-2,4-dion
58) 3-Allyl-1,3,8-triaza-1,7,7,9,9-pentamethylspiro[4.5]decan-2,4-dion
59) 3-Glycidyl-1,3,8-triaza-7,7,8,9,9-pentamethylspiro[4.5]decan-2,4-dion
60) 1,3,7,7,8,9,9-Heptamethyl-1,3,8-triazaspiro[4.5]decan-2,4-dion
61) 2-Iso-propyl-7,7,9,9-tetramethyl-1-oxa-3,8-diaza-4-oxo-spiro[4.5]decan
62) 2,2-Dibutyl-7,7,9,9-tetramethyl-1-oxa-3,8-diaza-4-oxo-spiro-[4.5]decan
63) 2,2,4,4-Tetramethyl-7-oxa-3,20-diaza-21-oxo-dispiro[5.1.11.2]-heneicosan
64) 2-Butyl-7,7,9,9-tetramethyl-1-oxa-4,8-diaza-3-oxo-spiro-[4,5]decan
65) 8-Acetyl-3-dodecyl-1,3,8-triaza-7,7,9,9-tetramethylspiro[4,5]-decan-2,4-dion

oder die Verbindungen der folgenden Formeln :

66)

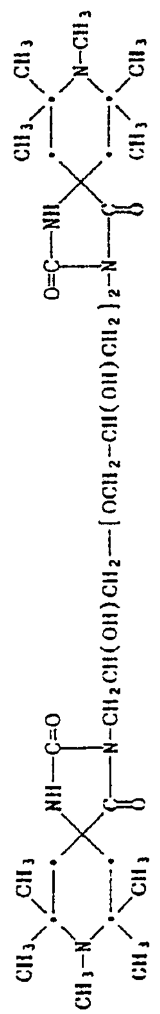

67)

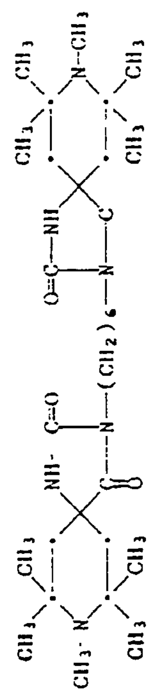

68)

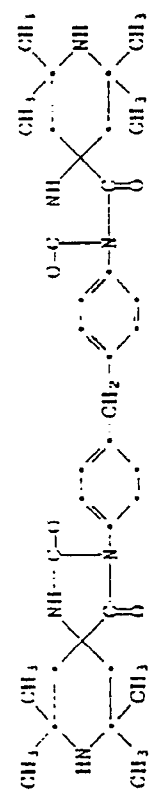

69)

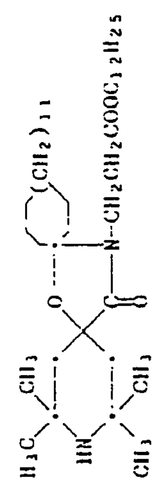

e) Verbindungen der Formel VII,

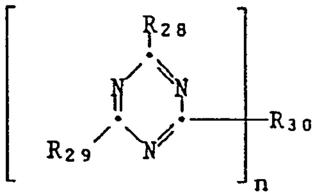

VII

worin n die Zahl 1 oder 2 ist und $R_{28}$ eine Gruppe der Formel

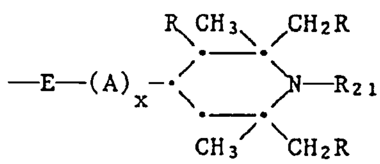

bedeutet, worin R und $R_{21}$ die unter a) angegebene Bedeutung haben, E —O— oder —$NR_{21}$— ist, A $C_2$-$C_6$-Alkylen oder —$(CH_2)_3$-O— und x die Zahlen O oder 1 bedeuten, $R_{29}$ gleich $R_{28}$ oder eine der Gruppen —$NR_{31}R_{32}$, —$OR_{33}$, —$NHCH_2OR_{33}$ oder —$N(CH_2OR_{33})_2$ ist, $R_{30}$, wenn n = 1 ist, gleich $R_{28}$ oder $R_{29}$, und wenn n = 2 ist, eine Gruppe —E-B-E— ist, worin B gegebenenfalls durch —$N(R_{31})$— unterbrochenes $C_2$-$C_6$-Alkylen bedeutet, $R_{21}$ $C_1$-$C_{12}$-Alkyl, Cyclohexyl, Benzyl oder $C_1$-$C_4$-Hydroxyalkyl oder eine Gruppe der Formel

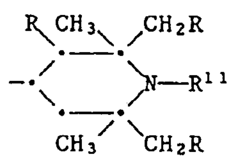

ist, $R_{32}$ $C_1$-$C_{12}$ Alkyl, Cyclohexyl, Benzyl, $C_1$-$C_4$ Hydroxyalkyl und $R_{33}$ Wasserstoff, $C_1$-$C_{12}$ Alkyl oder Phenyl bedeuten oder $R_{31}$ und $R_{32}$ zusammen $C_4$-$C_5$-Alkylen oder -Oxaalkylen, beispielsweise

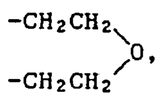 oder eine Gruppe der Formel 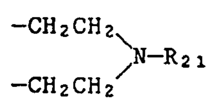

sind oder auch $R_{31}$ und $R_{32}$ jeweils eine Gruppe der Formel

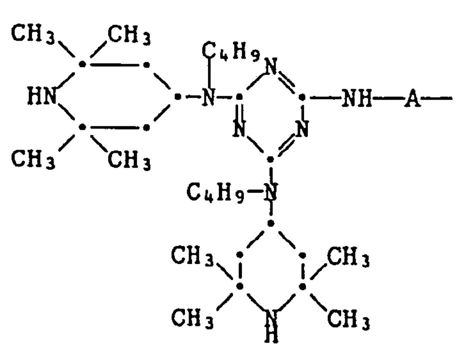

bedeuten.

Bedeuten etwaige Substituenten $C_1$-$C_{12}$-Alkyl, so stellen sie beispielsweise Methyl, Ethyl, n-Propyl, n-Butyl, sek.-Butyl, tert.-Butyl, n-Hexyl, n-Octyl, 2-Ethylhexyl, n-Nonyl, n-Decyl, n-Undecyl oder n-Dodecyl dar.

Bedeuten etwaige Substituenten $C_1$-$C_4$-Hydroxyalkyl, so stellen sie z.B. 2-Hydroxyethyl, 2-Hydroxypropyl,

3-Hydroxypropyl, 2-Hydroxybutyl oder 4-Hydroxybutyl dar.

Bedeutet A $C_2$-$C_6$ Alkylen, so stellt es beispielsweise Ethylen, Propylen, 2,2-Dimethylpropylen, Tetramethylen oder Hexamethylen dar.

Stellen $R_{31}$ und $R_{32}$ zusammen $C_4$-$C_5$-Alkylen oder Oxaalkylen dar, so bedeutet dies z.B. Tetramethylen, Pentamethylen oder 3-Oxapentamethylen.

Beispiele für Polyalkylpiperidin-Verbindungen dieser Klasse sind die Verbindungen der folgenden Formeln :

70)

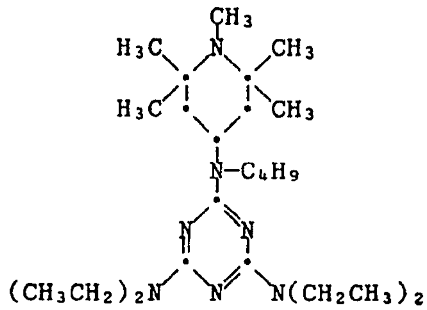

71)

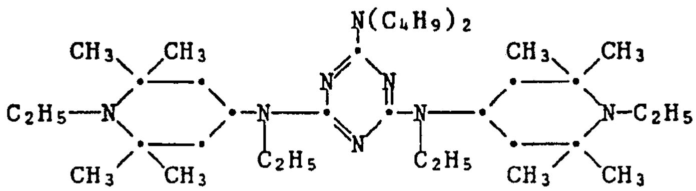

72)

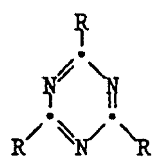

mit R =

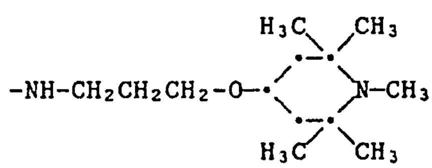

73)

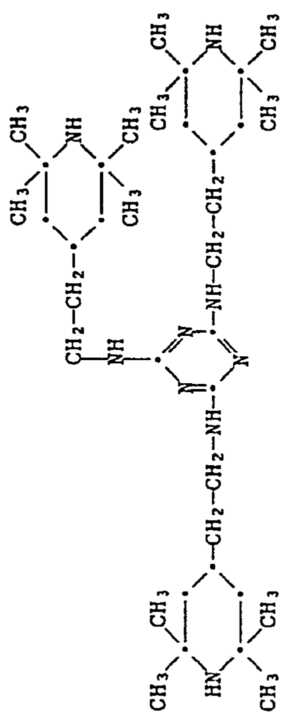

74)

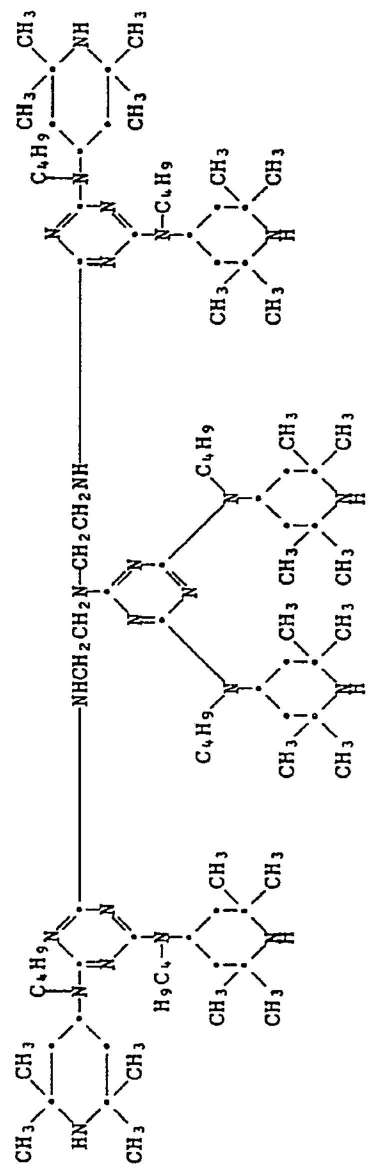

75) $R{-}NH{-}(CH_2)_3{-}N(R){-}(CH_2)_2{-}N(R){-}(CH_2)_3{-}NH{-}R$

mit R =

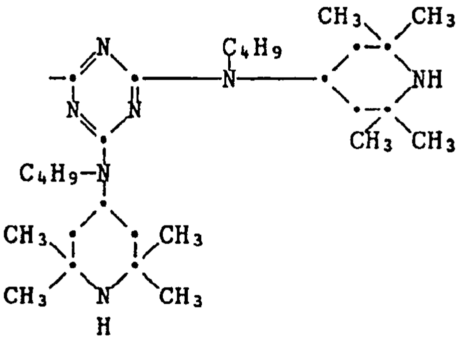

76) $R{-}NH{-}(CH_2)_3{-}N(R){-}(CH_2)_2{-}N(R){-}(CH_2)_3{-}NH{-}R$

mit R =

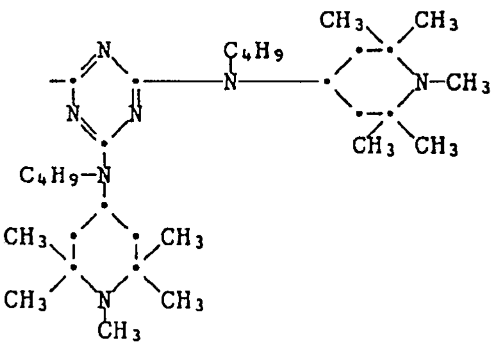

77) $R{-}N(CH_3){-}(CH_2)_3{-}N(R){-}(CH_2)_2{-}N(R){-}(CH_2)_3{-}N(CH_3){-}R$

mit R =

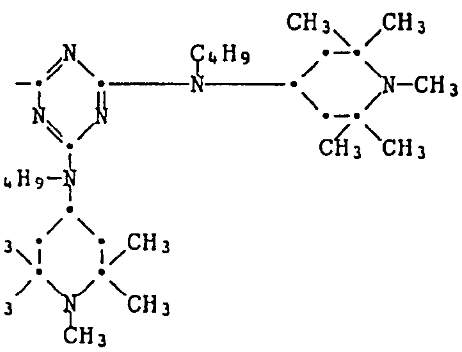

78) 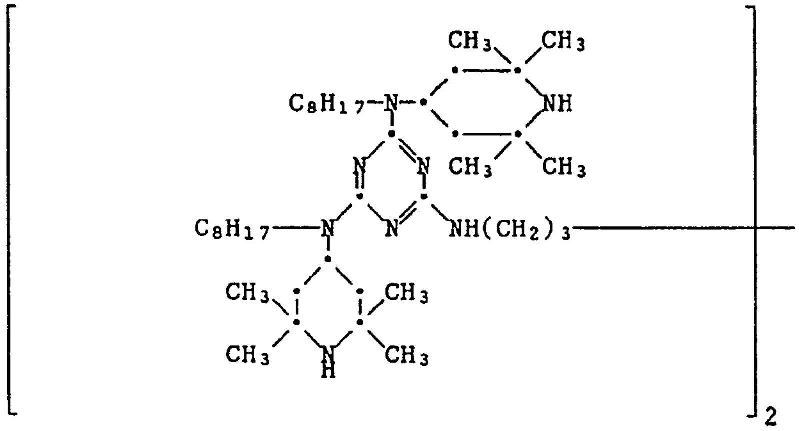

79) 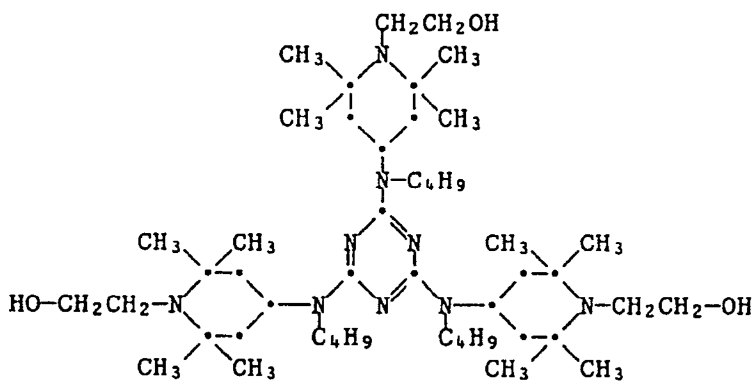

(80) 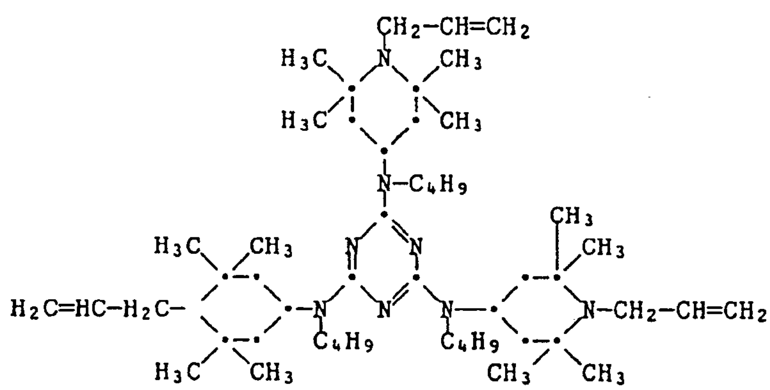

f) Oligomere oder polymere Verbindungen, deren wiederkehrende Struktureinheit einen 2,2,6,6-Tetraalkylpiperidinrest der Formel (I) enthält, insbesondere Polyester, Polyäther, Polyamide, Polyamine, Polyurethane, Polyharnstoffe, Polyaminotriazine, Poly(meth)acrylate, Poly(meth)acrylamide und deren Copolymere, die solche Reste enthalten.

Beispiele für 2,2,6,6-Polyalkylpiperidin-Lichtschutzmittel dieser Klasse sind die Verbindungen der folgenden Formeln, wobei m eine Zahl von 2 bis etwa 200 bedeutet.

81)

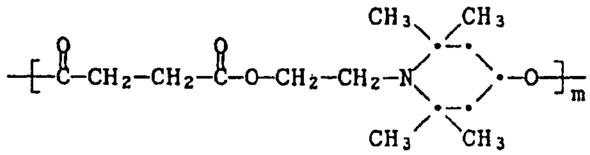

82)

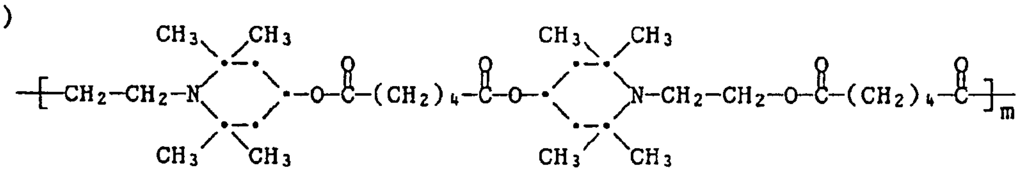

83)

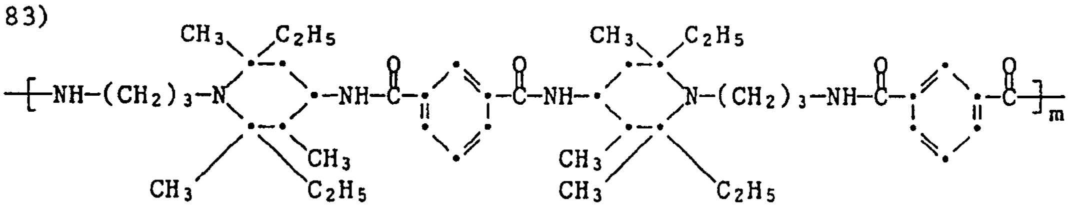

84)

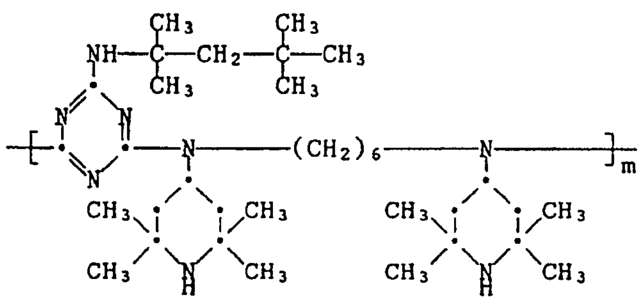

85)

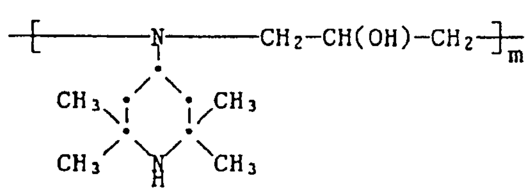

86)

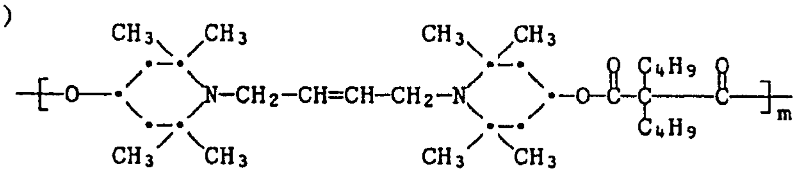

87)

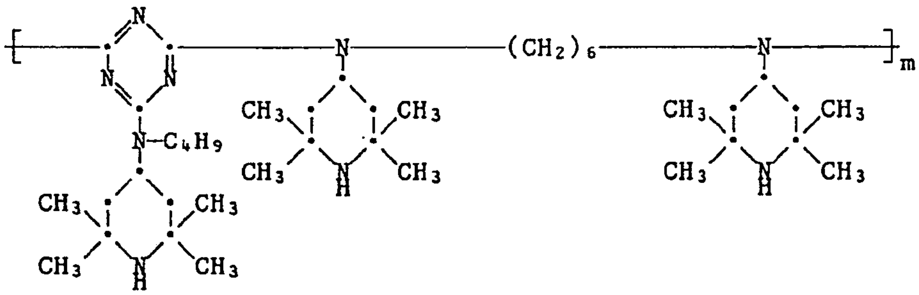

88)

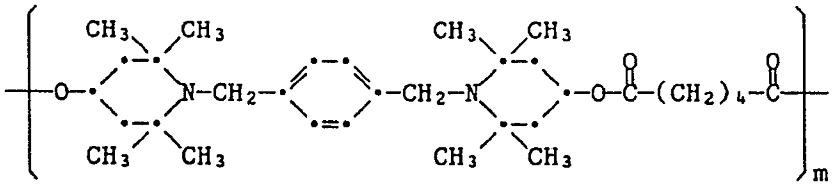

89)

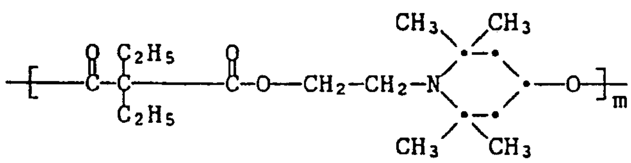

90)

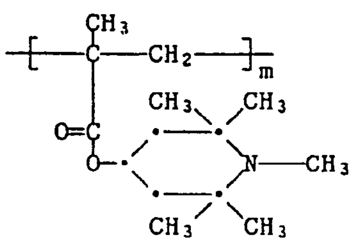

91)

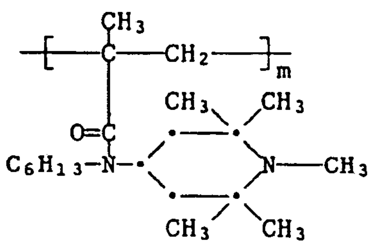

92)

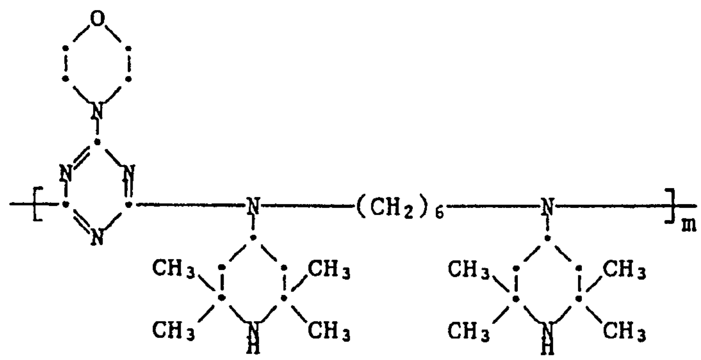

93)

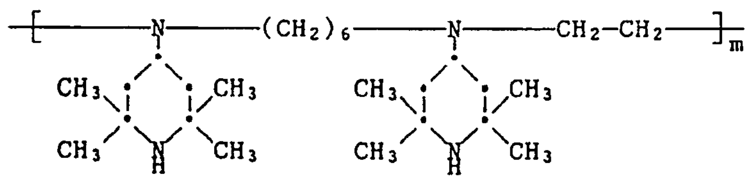

94)

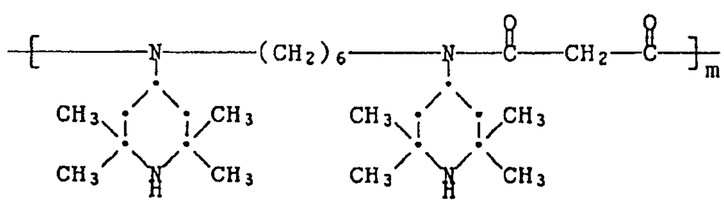

Von diesen Verbindungsklassen sind die Klassen a), d), e) und f) besonders geeignet, insbesondere die Verbindungen Nr. 10, 13, 14, 23, 24, 28, 29, 63, 65, 75, 77, 81, 84, 92 und 93.

Die erfindungsgemässen Materialien können neben den Verbindungen der Formel (1), worin X $CH_2$, NH, oder S ist bzw. neben den Kombinationen von Verbindungen der Formel (1) mit X = $CH_2$, NH, O oder S mit gehinderten Aminen auch weitere übliche Stabilisatoren und Zusätze enthalten, wie z.B. Antioxidantien, weitere Lichtschutzmittel, Metalldesaktivatoren, Verarbeitungsstabilisatoren, Nukleierungsmittel, Füllstoffe und andere Zusätze. Beispiele für derartige zusätzliche Inhaltsstoffe sind :

1. Antioxidantien

1.1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert.butyl-4-methoxymethylphenol, 2,6-Di-nonyl-4-methylphenol.

1.2. Alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Diphenyl-4-octadecyloxyphenol.

1.3. Hydroxylierte Thiodiphenylether, z.B. 2,2'-Thio-bis-(6-tert.-butyl-4-methylphenol), 2,2'-Thio-bis-(4-octylphenol), 4,4'-Thio-bis-(6-tert.butyl-3-methylphenol), 4,4'-Thio-bis-(6-tert.butyl-2-methylphenol).

1.4. Alkyliden-Bisphenole, z.B. 2,2'-Methylen-bis-(6-tert.butyl-4-methylphenol), 2,2'-Methylen-bis-(6-tert.butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 2,2'-Methylen-bis-[6-(α-methylbenzyl)-4-nonylphenol), 2,2'-Methylen-bis-[6-(α,α-dimethylbenzyl)-4-nonylphenol], 4,4'-Methylen-bis-(2,6-di-tert.-butylphenol), 4,4'-Methylen-bis-(6-tert.butyl-2-methylphenol), 1,1-Bis-(5-tert.butyl-4-hydroxy-2-methylphenyl)-butan, 2,6-Bis-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methylphenyl)-3-n-dodecylmercaptobutan, Ethylenglycol-bis-[3,3-bis-(3'-tert.-butyl-4'-hydroxyphenyl)-butyrat], Bis-(3-tert.butyl-4-hydroxy-5-methylphenyl)-dicyclopentadien, Bis-[2-(3'-tert.butyl-2'-hydroxy-5'-methylbenzyl)-6-tert.butyl-4-methyl-phenyl]-terephthalat.

1.5 Benzylverbindungen, z.B. 1,3,5-Tris-(3,5-di-tert.butyl-4-hydroxybenzyl)-2,4,6-trimethylbenzol, Bis-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl)dithiol-terephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, Ca-Salz des 3,5-Di-tert.butyl-4-hydroxybenzyl-phosphonsäure-monoethylester, 1,3,5-Tris-(3,5-dicyclohexyl-4-hydroxybenzyl)isocyanurat.

1.6. Acylaminophenole, z.B. 4-Hydroxy-laurinsäureanilid, 4-Hydroxystearinsäureanilid, 2,4-Bis-(octylmercapto)-6-(3,5-di-tert.butyl-4-hydroxyanilino)-s-triazin, N-(3,5-di-tert.butyl-4-hydroxyphenyl)-carbaminsäureoctylester.

1.7. Ester der β-(3,5-Di-tert.butyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid.

1.8. Ester der β-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.

1.9. Ester der β-(3,5-Dicyclohexyl-4-hydroxyphenyl)-propionsäure mit ein-oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid.

1.10. Amide der β-(3,5-Di-tert.butyl-4-hydroxyphenyl)-propionsäure, wie z.B. N,N'-Bis-(3,5-di-tert.butyl-4-hydroxyphenylpropionyl)-hexamethylendiamin, N,N'-Bis-(3,5-di-tert.butyl-4-hydroxyphenylpropionyl)-trimethylendiamin, N,N'-Bis-(3,5-di-tert.butyl-4-hydroxyphenylpropionyl)-hydrazin.

2. UV-Absorber und Lichtschutzmittel

2.1. 2-(2'-Hydroxyphenyl)-benztriazole, wie z.B. das 5'-Methyl—, 3',5'-Di-tert.butyl—, 5'-tert.Butyl—, 5'-(1,1,3,3-Tetramethylbutyl)—, 5-Chlor-3',5'-di-tert.butyl—, 5-Chlor-3'-tert.butyl-5'-methyl—, 3'-sec.Butyl-5'-tert.butyl, 4'-Octoxy—, 3',5'-Di-tert.amyl—, 3',5'-Bis-(α,α-dimethylbenzyl)-Derivat.

2.2. 2-Hydroxybenzophenone, wie z.B. das 4-Hydroxy—, 4-Methoxy—, 4-Octoxy—, 4-Decyloxy—, 4-Dodecyloxy—, 4-Benzyloxy—, 4,2',4'-Trihydroxy—, 2'-Hydroxy-4,4'-dimethoxy-Derivat.

2.3. Ester von gegebenenfalls substituierten Benzoesäuren, wie z.B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxyzimtsäure-methylester, N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1 : 1- oder der 1 : 2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Oxalsäurediamide, wie z.B. 4,4'-Di-octyloxy-oxanilid, 2,2'-Di-octyloxy-5,5'-di-tert.butyl-oxanilid, 2,2'-Di-dodecyloxy-5,5'di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von o- und p-Methoxy- sowie von o- und p-Ethoxy-di-substituierten Oxaniliden.

2.7. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin,

2-(2-Hydroxy-4-dodecyloxyphenyl)-4,6-bis(2,4-dimethylphenyl)-1,3,5-triazin.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert.butyl-4-hydroxyphenylpropionyl)-hydrazin, 3-Salicyloylamino-1,2,4-triazol, Bis-(benzyliden)-oxalsäuredihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit, 3,9-Bis-(2,4-di-tert.butylphenoxy)-2,4,8,10-tetraoxa-3,9-diphosphaspiro[5.5]undecan.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrittetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

Von den organischen Materialien, die erfindungsgemäss gegen Lichtschädigung geschützt werden können, sind organische Polymere hervorzuheben, insbesondere synthetische Polymere. Bevorzugt werden dabei thermoplastische Kunststoffe und insbesondere Lackzusammensetzungen geschützt, wobei bei letzteren insbesondere der polymere Binder gegen Lichteinfluss stabilisiert wird.

Lackzusammensetzungen bzw. Lacke, welche die Verbindungen der Formel (1) enthalten, können z.B. pigmentierte oder unpigmentierte Lacke oder Metalleffektlacke sein. Sie können ein organisches Lösungsmittel enthalten oder lösungsmittelfrei sein oder wässrige Lacke sein.

Die Lacke können als Bindemittel mindestens eines der vorhin aufgeführten Polymeren enthalten. Beispiele für Lacke mit speziellen Bindemitteln sind die folgenden :

1. Lacke auf Basis von kalt- oder heiss-vernetzbaren Alkyd-, Acrylat-, Polyester-, Epoxid- oder Melaminharzen oder Mischungen solcher Harze, gegebenenfalls mit Zusatz eines sauren Härtungskatalysators ;

2. Zweikomponenten-Polyurethanlacke auf Basis von hydroxylgruppenhaltigen Acrylat-, Polyester- oder Polyetherharzen und aliphatischen oder aromatischen Polyisocyanaten ;

3. Einkomponenten-Polyurethanlacke auf Basis von blockierten Polyisocyanaten, die während des Einbrennens deblockiert werden ;

4. Zweikomponentenlacke auf Basis von (Poly)ketiminen und aliphatischen oder aromatischen Polyisocyanaten ;

5. Zweikomponentenlacke auf Basis von (Poly)ketiminen und einem ungesättigten Acrylatharz oder einem Polyacetoacetatharz oder einem Methylacrylamidoglykolat-methylester ;

6. Zweikomponentenlacke auf Basis von carboxyl- oder aminogruppenhaltigen Polyacrylaten und Polyepoxiden ;

7. Zweikomponentenlacke auf Basis von anhydridgruppenhaltigen Acrylatharzen und einer Polyhydroxy- oder Polyaminokomponente ;

8. Zweikomponentenlacke auf Basis von (Poly)oxazolidinen und anhydridgruppenhaltign Acrylatharzen oder ungesättigten Acrylatharzen oder aliphatischen oder aromatischen Polyisocyanaten.

9. Zweikomponentenlacke auf Basis von ungesättigten Polyacrylaten und Polymalonaten ;

10. thermoplastische Polyacrylatlacke auf Basis von thermoplastischen Acrylatharzen oder fremdvernetzenden Acrylatharzen in Kombination mit veretherten Melaminharzen ;

11. Lacksysteme auf Basis von siloxanmodifizierten Acrylatharzen ; und

12. Lacksysteme auf Basis von fluormodifizierten Acrylatharzen.

Die Lacke können auch strahlenhärtbare Lacke sein. In diesem Fall besteht das Bindemittel aus monomeren oder oligomeren Verbindungen, die ethylenische Doppelbindungen enthalten und durch Bestrahlung mit aktinischem Licht oder mit Elektronenstrahlen in eine vernetzte hochmolekulare Form übergehen. Meist handelt es sich hierbei um ein Gemisch solcher Verbindungen.

Die Lacke können als Einschicht- oder Zweischichtlacke appliziert werden, wobei die erfindungsgemässen Stabilisatoren vorzugsweise der obersten Schicht zugesetzt werden.

Die Lacke können auf die Substrate (Metall, Plastik, Holz etc.) nach den üblichen Verfahren aufgebracht werden, beispielsweise durch Streichen, Besprühen, Giessen, Tauchen oder Elektrophorese. Besonders bevorzugt sind Lacke, die auf Automobile appliziert werden können (Autolacke).

Die Verbindungen der Formel (1) können nach üblichen, bekannten Methoden in Lacke bzw. Lackzusammensetzungen sowie andere lichtempfindliche organische Materialien eingearbeitet werden. Die Menge des Zusatzes von UV-Absorber hängt vom organischen Material und den Anforderungen an dessen Stabilität ab. Im allgemeinen setzt man, bezogen auf das Material, 0,01 bis 5 Gew.%, insbesondere 0,02 bis 3 Gew.% UV-Absorber zu.

Die Komponenten von Stabilisatorkombinationen können einzeln oder als Gemisch dem organischen Material zugesetzt werden. Der Zusatz der Verbindungen der Formel (1) und gegebenenfalls weiterer Stabilisatoren erfolgt vorzugsweise vor oder während der Formgebung des Materials, sofern es sich z.B. um thermoplastische Kunststoffe handelt. Die Zugabe kann jedoch auch bereits während der Polymerisation oder vor dieser zu den Ausgangsmonomeren erfolgen. Im Fall von Lackzusammensetzungen werden die erfindungsgemäss einsetzbaren Stabilisatoren insbesondere zur Lackformulierung oder zu Teilen der Lackformulierung vor der Applikation zugemischt.

Die erfindungsgemässen Verbindungen der Formel (1), worin X S ist, sowie auch die erfindungsgemäss verwendeten Verbindungen der Formel (1), worin X $CH_2$, NH oder O ist, erweisen sich als wirksame UV-Absorber, die sich durch eine hohe Lichtechtheit auszeichnen. Sie eignen sich deshalb zum dauerhaften Schutz lichtempfindlicher organischer Materialien vor schädigenden Lichteinflüssen.

Die Verbindungen der Formel (1) lassen sich nach an sich bekannten Methoden herstellen. Beispielsweise kann durch Erhitzen eines Gemisches aus den Verbindungen der Formeln

(2) 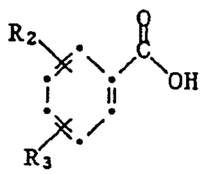 und (3) 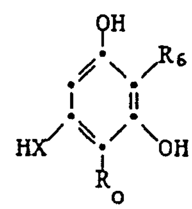 ,

worin $R_o$, $R_2$, $R_3$ und $R_6$ die angegebene Bedeutung haben, und X $CH_2$, NH, O oder S ist, die Zwischenverbindung der Formel

(4) 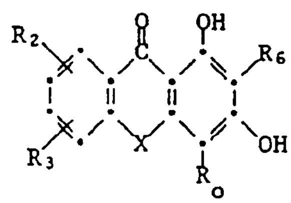

erhalten werden, welche sich durch Umsetzung mit beispielsweise $R_1$-Cl oder $R_1$-Br, worin $R_1$ die angegebene Bedeutung hat, in die entsprechende Verbindung der Formel (1) überführen lässt.

Die folgenden Beispiele erläutern die Erfindung weiter. Angaben in Teilen oder Prozenten beziehen sich — sofern nicht anders angegeben — auf das Gewicht.

Herstellungsbeispiele :

Beispiel 1 : 1-Hydroxy-3-octoxy-xanthon 22,8 g 1,3-Dihydroxyxanthon (hergestellt nach Grover, J. Chem. Soc. 1955, 3982) werden in 250 ml Methyläthylketon mit 15,2 g $K_2CO_3$ und 21,2 g 1-Bromoctan 15 Stunden am Rückfluss gekocht. Nach dem Abkühlen wird das Salz abfiltriert und die Methyläthylketonlösung am Vakuum eingedampft. Durch Kristallisation des Rückstandes aus Heptan erhält man das 1-Hydroxy-3-octoxy-xanthon mit einem Schmelzpunkt von 98°C.

Beispiel 2 : 22,8 g 1,3-Dihydroxyxanthon (hergestellt nach Grover, J. Chem. Soc. 1955, 3982) werden in 300 ml absolutem Aethanol vorgelegt. Dazu gibt man langsam, in Portionen, 11,2 g Kalium-tert.-butylat und dann 0,5 g Kaliumjodid. Bei Raumtemperatur werden in ca. 15 Minuten 12,3 g Chloressigsäure-äthylester zugetropft und das Reaktionsgemsich anschliessend 6 Stunden am Rückfluss erhitzt. Man giesst in 500 ml Wasser und säuert mit 20 ml Eisessig an. Der entstandene Niederschlag wird abgenutscht und getrocknet. Durch Umkristallisieren aus Hexan erhält man die Verbindung der Formel

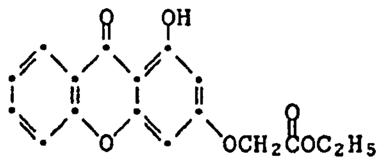

mit einem Schmelzpunkt von 165°C.

Beispiel 3 : 22,8 g 1,3-Dihydroxyxanthon (hergestellt nach Grover, J. Chem. Soc. 1955, 3982) werden in 200 ml Xylol auf 120°C erwärmt. Nach der Zugabe von 1,5 g Tetrabutylammoniumbromid werden in ca. 10 Minuten 20,5 g 2-Aethylhexyl-glycidyläther zugetropft und das Reaktionsgemisch dann 24 Stunden bei 120°C gerührt. Man gibt dann 5 g Tansil AC zur Reaktionslösung, rührt 5 Minuten bei 110°C und filtriert heiss. Das Filtrat wird eingedampft, der Rückstand in 300 ml Hexan aufgenommen und 50 g Kieselgel filtriert. Nach dem Eindampfen erhält man die Verbindung der Formel

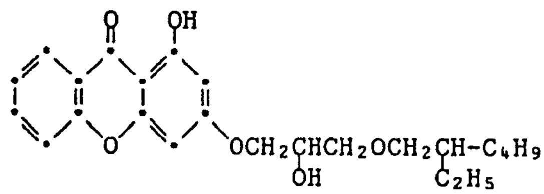

in Form eines gelblichen Harzes, das langsam kristallisiert. Der Schmelzpunkt beträgt 56 bis 58°C.

Anwendungsbeispiel 1 :

Die erfindungsgemässen UV-Absorber werden in einem 2-Schicht Metallic Lack geprüft.
Es wird ein Klarlack der folgenden Zusammensetzung hergestellt :

| | |
|---|---|
| Uracron® 2263 XB (50 %) | 59,2 Teile |
| Cymel® 327 (90 %) | 11,6 Teile |
| Baysilon® A (1 % in Xylol) | 1,0 Teile |
| Butylglykolacetat | 5,5 Teile |
| Xylol | 19,4 Teile |
| Butanol | 3,3 Teile |
| | 100,0 Teile |

Dazu wird 1 Teil der Verbindung nach Herstellungsbeispiel 1, vorgelöst in 10 Teilen Xylol, zugegeben.

Dieser Klarlack wird mit einem Gemisch aus Xylol (13 Teile), Butanol (6 Teile) und Butylglykolacetat (1 Teil) auf Spritzfähigkeit verdünnt und auf ein vorbereitetes Aluminiumblech (coil coat-beschichtet, Automobilfüller, silbermetallic Basislack-auf Basis Polyester/Celluloseacetatobutyrat/Melaminharz) gespritzt und bei 130°C 30 Minuten eingebrannt. Es resultiert eine Trockenschichtdicke von 40 bis 50 µm Klarlack. Vergleichsweise sind ein gleichermassen vorbereitetes Aluminiumblech mit dem oben definierten Klarlack, der jedoch frei von UV-Absorber ist, beschichtet.

Die so erhaltenen Proben werden nach Bewitterung auf Glanzhaltung geprüft. Im Vergleich zur Probe ohne UV-Absorber zeigen die erfindungsgemäss stabilisierten Proben eine deutlich bessere Bewitterungsstabilität.

Vergleichbar gute Eigenschaften zeigen auch Lacke, die neben den genannten UV-Absorbern ein sterisch gehindertes Amin der Formel

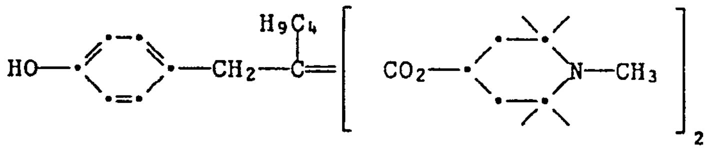

,

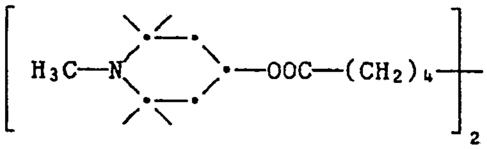

oder

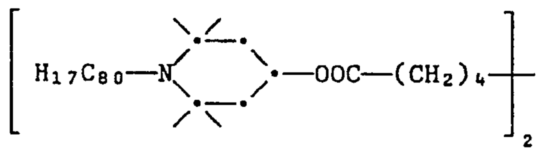

enthalten.

Die Ergebnisse sind in der folgenden Tabelle 1 zusammengestellt.

Tabelle 1:

Bewertung der Glanzhaltung nach Bewitterung gemäss DIN 67530 (20° Glanz)

| UV-Absorber | 20° Glanz nach 0 | 400 | 800 | 1200 Stunden |
|---|---|---|---|---|
| keiner | 86 | 75 | 45 | 16 |
| gemäss Beispiel 1 | 84 | 78 | 74 | 81 |
| gemäss Beispiel 2 | 85 | 75 | 68 | 74 |
| gemäss Beispiel 3 | 86 | 80 | 80 | 80 |
| gemäss Formel (5) | 85 | 73 | 70 | 74 |

Formel (5)

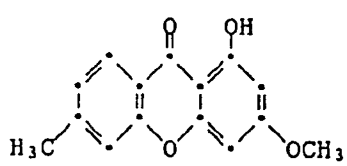

Zyklus : 8 Stunden UV (UVB-313), 70°C
4 Stunden Kond., 50°C (UVCON)

Anwendungsbeispiel 2 :

Man wiederholt Anwendungsbeispiel 1, setzt aber als zusätzlichen UV-Absorber 0,5 Teile der Verbindung der Formel

(6)

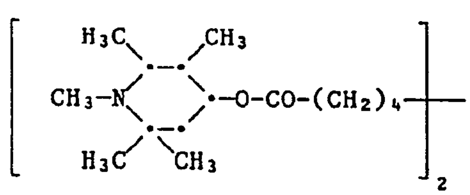

ein. Die Ergebnisse sind in Tabelle 2 enthalten.

Tabelle 2:

Bewertung der Glanzhaltung nach Bewitterung gemäss DIN 67530 (20° Glanz)

| UV-Absorber | 20° Glanz nach 0 | 400 | 800 | 1200 Stunden |
|---|---|---|---|---|
| keiner | 86. | 71 | 52 | 36 |
| 1 Teil gemäss Beispiel 1 + 0,5 Teile (6) | 86 | 70 | 61 | 56 |
| 1 Teil gemäss Beispiel 3 + 0,5 Teile (6) | 86 | 74 | 66 | 57 |

Zyklus: VDA-Methode C (Xenotest 1200)

**Patentansprüche**

1. Lichtempfindliches organisches Material, enthaltend als UV-Absorber mindestens eine Verbindung der Formel

(1) 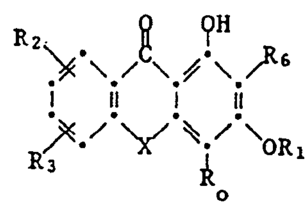

worin
X $CH_2$, NH oder S, $R_o$ Wasserstoff oder ein Rest der Formel $-(CH_2)_nCO_2R$, worin n 1 oder 2 und R Alkyl mit 1 bis 18 Kohlenstoffatomen oder $-(CH_2CH_2O)_mH$ ist, worin m 1 bis 12 ist, $R_1$ Wasserstoff, Alkyl mit 1 bis 12 Kohlenstoffatomen, mit Hydroxyl substituiertes und/oder mit Sauerstoff unterbrochenes Alkyl mit 2 bis 18 Kohlenstoffatomen, Alkenyl mit 2 bis 12 Kohlenstoffatomen, $-COR_4$, worin $R_4$ Alkyl mit 1 bis 12 Kohlenstoffatomen, Alkenyl mit 2 bis 12 Kohlenstoffatomen,

$$-(CH_2)_y-\overset{O}{\overset{\|}{C}}R_5 \text{ oder } -O-CH_2CH(OH)CH_2O-\overset{O}{\overset{\|}{C}}R_5 \text{ ist, oder } R_1 \; -(CH_2)_yO-\overset{O}{\overset{\|}{C}}R_5 \text{ ist,}$$

worin $R_5$ Alkyl mit 1 bis 12 Kohlenstoffatomen oder Alkenyl mit 2 bis 12 Kohlenstoffatomen und y 1 bis 12 ist,
$R_2$ und $R_3$ unabhängig voneinander Wasserstoff, Alkyl mit 1 bis 12 Kohlenstoffatomen, Alkenyl mit 2 bis 12 Kohlenstoffatomen, $-OR_1$, worin $R_1$ die angegebene Bedeutung hat, oder Chlor sind, und
$R_6$ Wasserstoff oder Alkyl und 1 bis 4 Kohlenstoffatomen ist.

2. Material nach Anspruch 1, worin $R_o$ ein Rest der Formel $-CH_2CH_2CO_2R$, worin R Alkyl mit 1 bis 18 Kohlenstoffatomen oder $-(CH_2CH_2O)_mH$ ist, worin m 4 bis 8 ist.

3. Material nach Anspruch 1, worin $R_o$ ein Rest der Formel $-CH_2CH_2CO_2CH_3$ ist.

4. Material nach Anspruch 1, worin in der Verbindung der Formel (1) $R_2$ und $R_3$ Wasserstoff sind.

5. Material nach Anspruch 1, worin in der Verbindung der Formel (1) $R_1$ Wasserstoff, Alkyl mit 4 bis 8 Kohlenstoffatomen, mit Hydroxyl substituiertes und/oder Sauerstoff unterbrochenes Alkyl mit 2 bis 12 Kohlenstoffatomen, Alkenyl mit 4 bis 12 Kohlenstoffatomen oder $-COR_4$ ist, worin $R_4$ Alkyl mit 4 bis 8 Kohlenstoffatomen ist.

6. Material nach Anspruch 5, worin in der Verbindung der Formel (1) $R_1$ Wasserstoff, Alkyl oder Alkenyl mit je 4 bis 8 Kohlenstoffatomen ist.

7. Lichtempfindliches organisches Material, enthaltend als UV-Absorber mindestens eine Verbindung der Formel (1) nach Anspruch 1, worin X $CH_2$, NH, O oder S ist, in Kombination mit mindestens einem sterisch gehinderten Amin oder Hydroxyphenylbenztriazolderivat.

8. Material nach Anspruch 1 oder 7, dadurch gekennzeichnet, dass es ein Lack oder ein thermoplastisches Polymer ist.

9. Verfahren zum Schützen lichtempfindlicher organischer Materialien vor UV-Strahlung, dadurch gekennzeichnet, dass diese Materialien mit einer mindestens eine Verbindung der Formel (1) nach Anspruch 1, worin X $CH_2$, NH oder S ist, enthaltenden Schutzschicht versehen wird, oder dass in diese Materialien mindestens eine Verbindung der Formel (1) nach Anspruch 1, worin X $CH_2$, NH oder S ist, eingearbeitet wird.

10. Verfahren zum Schützen lichtempfindlicher organischer Materialien vor UV-Strahlung, dadurch gekennzeichnet, dass diese Materialien mit einer mindestens eine Verbindung der Formel (1) nach Anspruch 1, worin X $CH_2$, NH, O oder S ist, enthaltenen Schutzschicht versehen wird, oder dass in diese Materialien mindestens eine Verbindung der Formel (1) nach Anspruch 1, worin X $CH_2$, NH, O oder S ist, eingearbeitet wird, wobei die Verbindung der Formel (1) nach Anspruch 1 jeweils in Kombination mit mindestens einem sterisch gehinderten Amin oder Hydroxyphenylbenztriazolderivat verwendet wird.

11. Verbindungen der Formel (1) nach Anspruch 1, worin X S, $R_o$ Wasserstoff oder ein Rest der Formel $(CH_2)_nCO_2R$, worin n 1 oder 2 und n R Alkyl mit 1 bis 18 Kohlenstoffatomen oder $(CH_2CH_2O)_m$ H ist, worin m 1 bis 12 ist, $R_1$ Wasserstoff, Alkyl mit 1 bis 12 Kohlenstoffatomen, mit Hydroxyl substituiertes und/oder mit Sauerstoff unterbrochenes Alkyl mit 4 bis 18 Kohlenstoffatomen, Alkenyl mit 2 bis 12 Kohlenstoffatomen, $-COR_4$, worin $R_4$ Alkyl mit 1 bis 12 Kohlenstoffatomen, Alkenyl mit 2 bis 12 Kohlenstoffatomen,

$$-(CH_2)_y-\overset{O}{\overset{\|}{C}}R_5 \text{ oder } -O-CH_2CH(OH)CH_2O-\overset{O}{\overset{\|}{C}}R_5 \text{ ist, oder } R_1\ -(CH_2)_yO-\overset{O}{\overset{\|}{C}}R_5 \text{ ist,}$$

worin $R_5$ Alkyl mit 1 bis 12 Kohlenstoffatomen oder Alkenyl mit 2 bis 12 Kohlenstoffatomen und y 1 bis 12 ist,
$R_2$ und $R_3$ unabhängig voneinander Wasserstoff, Alkyl mit 1 bis 12 Kohlenstoffatomen, Alkenyl mit 2 bis 12 Kohlenstoffatomen, $-OR_1$, worin $R_1$ die angegebene Bedeutung hat, oder Chlor sind, und
$R_6$ Wasserstoff oder Alkyl mit 1 bis 4 Kohlenstoffatomen ist.

12. Verbindungen nach Anspruch 11, dadurch gekennzeichnet, dass $R_o$ ein Rest der Formel $-CH_2CH_2CO_2R$, worin R Alkyl mit 1 bis 8 Kohlenstoffatomen oder $(CH_2CH_2O)_m$ H ist, worin m 6 bis 8 ist.

13. Verbindungen nach Anspruch 11, dadurch gekennzeichnet, dass $R_o$ ein Rest der Formel $-CH_2CH_2CO_2CH_3$ ist.

14. Verbindungen nach Anspruch 11, dadurch gekennzeichnet, dass $R_2$ und $R_3$ Wasserstoff sind.

15. Verbindungen nach Anspruch 11, dadurch gekennzeichnet, dass $R_1$ Wasserstoff, Alkyl mit 4 bis 8 Kohlenstoffatomen, mit Hydroxyl substituiertes und/oder Sauerstoff unterbrochenes Alkyl mit 2 bis 12 Kohlenstoffatomen, Alkenyl mit 4 bis 12 Kohlenstoffatomen oder —$COR_4$ ist, worin $R_4$ Alkyl mit 4 bis 8 Kohlenstoffatomen ist.

**16.** Verbindungen nach Anspruch 15, dadurch gekennzeichnet, dass $R_1$ Wasserstoff, Alkyl oder Alkenyl mit je 4 bis 8 Kohlenstoffatomen ist.

**17.** Verfahren zur Herstellung der Verbindungen der Formel (1) nach Anspruch 1, worin X S und $R_o$ ein Rest der Formel —$OR_1$ ist, dadurch gekennzeichnet, dass man ein Gemisch aus den Verbindungen der Formeln

(2) 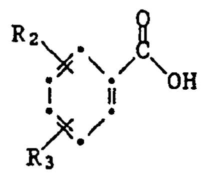 und (3) 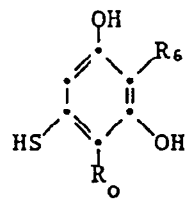 ,

worin $R_o$, $R_2$, $R_3$ und $R_6$ die in Anspruch 11 angegebene Bedeutung haben, erhitzt, die Zwischenverbindung der Formel

(4) 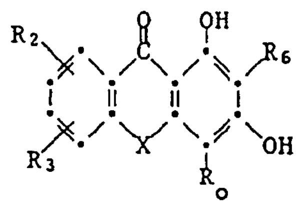

mit $R_1$-Cl oder $R_1$-Br, worin $R_1$ die in Anspruch 11 angegebene Bedeutung hat, zur entsprechenden Verbindung der Formel (1) umsetzt und isoliert.